# EUROPÄISCHE PATENTSCHRIFT

(11) **EP 2 838 875 B1**
(45) Veröffentlichungstag und Bekanntmachung des Hinweises auf die Patenterteilung: **25.05.2016**
(21) Anmeldenummer: 13715697.2
(22) Anmeldetag: 12.04.2013
(51) Int. Cl.: C07C 209/36, C07C 209/84, C07C 211/46, C07C 211/47

(54) **VERFAHREN ZUM VERBESSERTEN ANFAHREN DER REAKTION BEI DER HERSTELLUNG VON AROMATISCHEN AMINEN AUS NITROAROMATEN**
METHOD FOR IMPROVED STARTING THE REACTION WHEN PRODUCING AROMATIC AMINES FROM NITROAROMATICS
PROCÉDÉ DE DÉPART AMÉLIORÉ DE LA RÉACTION LORS DE LA FABRICATION D'AMINES AROMATIQUES À PARTIR DE NITRO-AROMATES

(30) Priorität: 16.04.2012 EP 12164325
(43) Veröffentlichungstag der Anmeldung: 25.02.2015
(73) Patentinhaber: Covestro Deutschland AG, 51373 Leverkusen (DE)
(72) Erfinder: MERKEL, Michael, 40223 Düsseldorf (DE); KNAUF, Thomas, 41542 Dormagen (DE); PETERS, Cliff Andre, 25724 Schmedeswurth (DE); SCHMIDT, Thorsten, 25704 Nindorf (DE)
(74) Vertreter: Levpat
(86) Internationale Anmeldenummer: PCT/EP2013/057717
(87) Internationale Veröffentlichungsnummer: WO 2013/156410

(56) Entgegenhaltungen:
- EP-A1- 0 212 602
- EP-A2- 0 944 578
- US-B1- 6 521 791

## Beschreibung

Die vorliegende Erfindung betrifft ein Verfahren zum verbesserten Anfahren der Reaktion bei der Herstellung von aromatischen Aminen aus Nitroaromaten, umfassend die Schritte des Bereitstellens eines Reaktors mit hierin enthaltenem Hydrierkatalysator, des Zuführens von aromatischen Nitroverbindungen und Wasserstoff in den Reaktor unter Kontaktierung des Hydrierkatalysators, wobei wenigstens der Wasserstoff mittels eines Verdichters in den Reaktor zugeführt wird und der Verdichter Betriebsflüssigkeit enthält, welche den Wasserstoff zumindest teilweise kontaktiert und des Regenerierens des Hydrierkatalysators durch Erwärmen und Kontaktieren mit Sauerstoff. Anschließend werden bestimmte (Betriebs-)Flüssigkeiten ausgetauscht.

Aromatische Amine sind wichtige Zwischenprodukte, die preiswert und in großen Mengen hergestellt werden müssen. Daher werden Produktionsaulagen für aromatische Amine in der Regel für sehr große Kapazitäten errichtet. Die hohe Produktivität dieser Anlagen ist durch sehr lange Reaktionszyklen und den störungsfreien Ablauf zwischen den An- und Abfahrvorgängen der Hydrierung zur Regeneration der eingesetzten Hydrierkatalysatoren gewährleistet.

Anilin ist wichtiges Zwischenprodukt z.B. zur Herstellung von Methylendiphenyldiisocyanat (MDI) und wird im großtechnischen Maßstab in der Regel durch katalytische Hydrierung von Nitrobenzol mit Wasserstoff hergestellt. Besonders bevorzugt sind Reaktionsführungen wie in GB 1 452 466 A1, EP 0 011 090 A1 oder EP 0 944 578 A2 (isotherme Fahrweise) und in EP 0 696 574 B1, EP 0 696 573 B1, EP 1 882 681 A1 (adiabate Fahrweise) beschrieben. Neben den genannten Verfahren mit stationären Katalysatorbetten sind auch solche mit fluidisierten Katalysatorbetten z. B. in DE 1114820 B, DE 1133394 B oder WO 2008/034770 A1 beschrieben.

Den beschriebenen adiabaten und isothermen Verfahren ist gemein, dass der Ausgangsstoff Nitrobenzol mit einem Überschuss an Wasserstoff umgesetzt wird.

Die Herstellung der aromatischen Amine erfolgt in Reaktionszyklen, weil die Katalysatoraktivität der Hydrierkatalysatoren stetig abnimmt.

Die Aktivität von gebrauchten Katalysatoren für die Hydrierung aromatischer Nitroverbindungen muss deshalb in periodischen Abständen wiederhergestellt werden. Dazu wird eine Regenerierung durchgeführt, indem kohlenstoffhaltige Ablagerungen vom Katalysator durch Abbrennen im Luftstrom entfernt werden. In anderen Ausführungsformen des Verfahrens wird der Abbrennstufe eine Waschstufe nachgeschaltet wie zum Beispiel in US 3,684,740 beschrieben. Danach kann der nächste Reaktionszyklus gestartet werden, indem die Hydrieranlage wieder angefahren wird. Auch Verfahren mit mehrmaliger Wäsche sind in WO 2012/013677 A1 beschrieben.

Allen aufgeführten Literaturstellen gemein ist, das sie den Anfahrprozess und seine Schwierigkeiten nicht beschreiben.

In einem Verfahren zur Hydrierung von Nitroverbindungen zu den entsprechenden Aminen gemäß EP 0 944 578 A2 (S. 2, Z. 1-20) wird auf das Anfahrprocedere eingegangen. Dort wird beschrieben, dass es sich verteilhaft auf die Raum-Zeit-Ausbeute auswirkt, die Belastung des Katalysators an eingesetzten aromatischen Nitroverbindungen kontinuierlich oder stufenweise innerhalb von 10 bis 1000 Stunden auf die maximale Belastung zu erhöhen.

US 6,521,791 B1 befasst sich mit einem Verfahren zur Regenerierung von Monolith-Katalysereaktoren, welche in Flüssigphasenhydrierungen insbesondere von Nitroaromaten Verwendung finden. Das Verfahren umfasst zwei Schritte, wobei zuerst ein oxidierendes und dann ein reduzierendes Gas durch den Monolith-Katalysereaktor geleitet wird.

Die Güte eines Verfahrens zur Hydrierung aromatischer Nitroverbindungen ist einerseits definiert durch den Gehalt des Produktes an unerwünschten Nebenprodukten der Reaktion. Andererseits ist die Güte eines Hydrierverfahrens dadurch definiert, dass der gesamte Prozess von Hydrierzyklus, Abfahren der Hydrierung, Regeneration des Hydrierkatalysators und das Anfahren des Hydrierprozesses ohne technischen Produktionsausfall betrieben werden kann.

Den beschriebenen Verfahren des Standes der Technik gelingt es zwar, aromatische Amine herzustellen, die einen geringen Gehalt an Nebenprodukten aufweisen, die also zum Beispiel nur zwischen 50 ppm und 300 ppm an Phenolen enthalten, jedoch zählen zu diesen Nebenprodukten immer auch Produkte, die durch Freisetzung von Ammoniak entstehen (Desaminierungsreaktion). Dieser Ammoniak kann im Reaktionssystem oder den Abgasleitungen mit anderen Verbindungen (zum Beispiel CO₂) reagieren und Niederschläge bilden. Eine Trennung ammoniakhaltiger Abgasströme und deren separate Entsorgung (zum Beispiel separate Einleitung in eine thermische Abluftreinigung) erfordert einen erhöhte Apparativen Aufwand und ist nicht immer vollständig möglich. Die möglichen Problematiken beim Anfahren eines Herstellungsprozesses von aromatischen Aminen werden übergangen.

Aufgabe der vorliegenden Erfindung war es daher ein Verfahren bereitzustellen, welches ein reibungsloses Anfahren der Hydrierreaktion gewährleistet, und welches nicht zum Entstehen von Ablagerungen in den Reaktionsapparaten und deren Peripherie führt, so dass es nicht zu einem Erzwungenem Abfahren der Anlage beim Anfahren oder nach kurzen Hydrierzyklen wegen Verstopfungen in den Reaktionsapparaten oder deren Peripherie kommt.

Erfindungsgemäß wird diese Aufgabe gelöst durch ein Verfahren zur Herstellung von aromatischen Aminen durch Hydrierung von aromatischen Nitroverbindungen, umfassend die Schritte:
A) Bereitstellen eines Reaktors mit hierin enthaltenem Hydrierkatalysator;
B) Zuführen von aromatischen Nitroverbindungen und Wasserstoff in den Reaktor unter Kontaktierung des Hydrierkatalysators, wobei wenigstens der Wasserstoff mittels eines Verdichters in den Reaktor zugeführt wird und der Verdichter Betriebsflüssigkeit enthält, welche den Wasserstoff zumindest teilweise kontaktiert;
C) Regenerieren des Hydrierkatalysators durch Erwärmen und Kontaktieren mit Sauerstoff.

Nach Schritt C) werden die Schritte D1) und/oder D2) durchgeführt:
D1) Zumindest teilweises Entfernen von im Reaktor und/oder in stromabwärts mit dem Reaktor fluidisch verbundenen Anlagenteilen befindlichen Flüssigkeiten, welche zumindest teilweise während Schritt C) im Reaktor und/oder in diesen Anlagenteilen anwesend waren;
D2) Zumindest teilweises Austauschen der Betriebsflüssigkeit des Verdichters, welche während Schritt C) im Verdichter und/oder in mit dem Verdichter verbundenen Rezirkulierungseinrichtungen anwesend war, gegen Betriebsflüssigkeit, welche während Schritt C) nicht im Verdichter und/oder in mit dem Verdichter verbundenen Rezirkulierungseinrichtungen anwesend war.

Überraschend wurde folglich gefunden, dass die Reaktionsräume und/oder deren Peripherie von Kohlendioxid zumindest teilweise befreit werden können, indem enthaltene Flüssigkeiten, die sich im Regenerationsprozess mit CO₂ gesättigt haben, durch frische Flüssigkeiten ausgetauscht werden. Das CO₂ entsteht hierbei wenigstens teilweise durch das Abbrennen von organischen Rückständen auf dem Katalysator.

Wenn die Reaktionsräume und/oder deren Peripherie von CO₂ befreit sind und die Betriebsflüssigkeit im Verdichter erneuert wird, ergeben sich die folgenden Vorteile für das Anfahrprocedere der Hydrierung:
i) Die Produktivität der Anlage erhöht sich, weil die Anlage nicht wegen Verstopfungen in den Reaktionsapparaten und deren Peripherie wie Abgasleitungen ausfällt.
ii) Die Energiekosten für wiederholtes Anfahren werden eingespart.
iii) Die Produktqualität leidet nicht wegen wiederholter Anfahrvorgänge.
iv) Reinigungskosten für das Entfernen von Niederschlägen aus den Reaktionsapparaten und deren Abgasleitungen entfallen.

Somit löst das erfindungsgemäße Verfahren die gestellte Aufgabe, indem Kohlenstoffdioxid vor dem Anfahren der Reaktion aus dem Reaktionssystem und dessen Peripherie (also zum Beispiel dem Kreisgasverdichter) entfernt wird, so dass es nicht mehr zur Bildung von Ablagerungen beitragen kann. Das Anfahren der Hydrierung und die anschließenden Aufarbeitung der entstandenen Anfahrware erfolgt somit technisch reibungslos ohne Ausfallzeiten mit nur einmalig eingeschränkter Endproduktqualität ohne den Zwang durch mehrfaches Anfahren mehr Ware verschneiden zu müssen.

Das Gleiche gilt selbstverständlich sinngemäß hinsichtlich in den Reaktorflüssigkeiten und/oder Betriebsflüssigkeiten des Verdichters gelösten Ammoniaks.

Das erfindungsgemäße Verfahren eignet sich generell für das Anfahren von Hydrierprozessen in denen mit der Bildung von Ammoniak gerechnet werden muss. Insbesondere eignet es sich für das Anfahren von Hydrierungen wie sie in EP 0 944 578 A2, EP 0 011 090 A1, EP 0 696 574 B1, EP 0 696 573 B1, EP 1 882 681 A1, GB 1 452 466 A1, DE 1114820 B, DE 1133394 B oder WO 2008/034 770 A1 beschrieben sind. Es eignet sich besonders, wenn die Katalysatoren, wie in US 3,684,740 beschrieben, regeneriert wurden.

Insbesondere eignet es sich für das erfindungsgemäße Verfahren, wenn der Katalysator katalytisch aktive Komponenten auf einem Aluminiumoxid-Träger mit einem mittleren Durchmesser der Aluminiumoxid-Partikel zwischen 1,0 mm und 7,0 mm und einer BET-Oberfläche von weniger als 20 m²/g enthält, und bei dem die aktiven Komponenten mindestens umfassen:
(a) 1 - 100 g/l Träger mindestens eines Metalls der Gruppen 8 bis 12 des Periodensystems der Elemente, und
(b) 0 - 100 g/l Träger mindestens eines Übergangsmetalls der Gruppen 4 bis 6 und 12 des Periodensystems der Elemente, sowie
(c) 0 - 100 g/l Träger mindestens eines Metalls der Hauptgruppenelemente der Gruppen 14 und 15 des Periodensystems der Elemente.

Die Gruppen des Periodensystems der Elemente werden in dieser Druckschrift gemäß der IUPAC-Empfehlung von 1986 gezählt.

Der Aluminiumoxidträger hat bevorzugt annähernd Kugelform und bevorzugt einen Durchmesser im Bereich von 1,0 mm bis 7,0 mm.

Hinsichtlich der Betriebsweise des Reaktors bevorzugt sind Reaktionsführungen wie in EP 0 944 578 A2 (isotherme Fahrweise) und in EP 0 696 574 B1, EP 0 696 573 B1, EP 1 882 681 A1 (adiabate Fahrweise) beschrieben.

Bevorzugte Reaktoren für einen isotherm betriebenen Reaktor sind thermostatisierte Rohr- oder Rohrbündelreaktoren. Geeignete Ausführungsformen solcher Reaktoren sind zum Beispiel in DE 2 201 528 A1, DE 2 207 166 A1, DE 198 06 810 A1, EP 1 439 901 A1, EP 1 569 745 A1, EP 1 590 076 A1, EP 1 587 612 A1, EP 1 586 370 A1, EP 1 627 678 A1 oder DE 202 006 014 116 U1 beschrieben.

Bevorzugte Reaktoren für einen adiabat betriebenen Reaktor sind die in DE 10 2006 035 203, Absätze [0030] bis [0033] beschriebenen.

Es ist möglich, dass der in dem Reaktor angeordnete Katalysator in einer radial durchströmten Filterkerze vorliegt. Dies kann zum Beispiel erreicht werden, indem der Katalysator in einem Korb gehalten wird, der aus zwei konzentrischen, zylindrischen Siebmänteln mit fluiddurchlässigen Wänden aufgebaut ist. Dabei weist ein Siebmantel einen größeren Radius auf als der andere, der auch als Zentralrohr bezeichnet wird, und der Raum zwischen den Siebmänteln ist der Reaktionsraum. Ein Boden dieses Hohlzylinders ist vorzugsweise komplett dicht verschlossen, während der andere nur bis an das Zentralrohr heran geschlossen ist, das an diesem Ende offen ist. Das Fluid kann nun in radialer Richtung von außen nach innen strömen und dann durch das Zentralrohr abgeführt werden. Alternativ kann das Fluid auch durch das Zentralrohr zugeführt werden und dann in radialer Richtung nach außen strömen, wo es dann abgeführt wird. Befindet sich dieser Reaktionsraum im gleichen Reaktor wie der isotherme Reaktionsraum, so ist er meist auf geeignete Weise mit dem Reaktorauslass verbunden.

Die Hydrierung der Nitroverbindungen erfolgt bevorzugt kontinuierlich und unter Rückführung nicht umgesetzten Wasserstoffs in die Reaktion.

Die vorliegende Erfindung wird anhand von Ausführungsformen weiter erläutert werden. Sie können beliebig miteinander kombiniert werden, sofern sich aus dem Zusammenhang nicht eindeutig das Gegenteil ergibt.

In einer Ausführungsform des erfindungsgemäßen Verfahrens ist der Verdichter ein Flüssigkeitsringverdichter. Ein Flüssigkeitsringverdichter wird häufig auch als Wasserringpumpe, Flüssigkeitsringpumpe, Flüssigringverdichter oder Flüssigringpumpe bezeichnet. Das Funktionsprinzip des Flüssigkeitsringverdichters basiert auf einem sternförmig, exzentrisch angeordneten Flügelrad/Laufrad, das sich im zylindrischen Gehäuse der Pumpe befindet. Die in dem Gehäuse enthaltene Betriebsflüssigkeit (im Normalfall Wasser oder Kondensat) bildet bei Rotation durch Zentrifugalkraft einen zum Gehäuse konzentrischen Flüssigkeitsring, welcher die Laufkammern abdichtet.

Die Betriebsflüssigkeit hat mehrere Funktionen. Sie soll abdichten, verdichten, kühlen und kondensieren. Die bei der Verdichtung anfallende Kompressions- und Kondensationswärme werden über die Betriebsflüssigkeit abgeführt. Diese wird dazu im Kreislauf gekühlt und rezirkuliert.

In einer weiteren Ausführungsform des erfindungsgemäßen Verfahrens ist die Betriebsflüssigkeit im Verdichter Wasser oder eine wässrige Lösung mit mehr als 90 Gewichts-% Wasser. Beispielsweise wird für einen Hydrierprozess von aromatischen Nitroverbindungen, der sich auszeichnet durch die Verwendung von Flüssigkeitsringpumpen als Wasserstoffkreisgasverdichter, die in dem Gehäuse enthaltene Flüssigkeit (bei der Hydrierung von Nitroaromaten ist die Flüssigkeit Wasser) vor dem Anfahren durch frische Flüssigkeit ersetzt.

In einer weiteren Ausführungsform des erfindungsgemäßen Verfahrens wird nach Schritt C) der Reaktor zusätzlich mit einem Inertgas befüllt oder gespült. Ein geeignetes Inertgas ist insbesondere Stickstoff.

In einer weiteren Ausführungsform des erfindungsgemäßen Verfahrens wird Schritt D1) durchgeführt, indem der Reaktor und/oder stromabwärts mit dem Reaktor fluidisch verbundenen Anlagenteile mit Wasser und/oder einer wässrigen Lösung mit mehr als 90 Gewichts-% Wasser (zum Beispiel Kondensat) gespült wird.

In einer weiteren Ausführungsform des erfindungsgemäßen Verfahrens beträgt in Schritt B) das molare Verhältnis von Wasserstoff zu Nitrogruppen der aromatischen Nitroverbindungen ≥ 3:1 bis ≤ 100:1. Bevorzugte Bereiche dieses Verhältnisses sind ≥ 3:1 bis ≤ 10:1 bei isothermer Prozeßführung beziehungsweise ≥ 60:1 bis ≤ 100:1 bei adiabater Prozessführung.

In einer weiteren Ausführungsform des erfindungsgemäßen Verfahrens werden in Schritt B) aromatische Nitroverbindungen gemäß der allgemeinen Formel (I) eingesetzt: 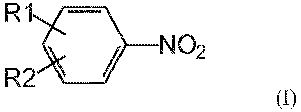 in der R1 und R2 unabhängig voneinander Wasserstoff, Methyl oder Ethyl bedeuten, wobei R2 weiterhin auch NO₂ bedeuten kann. Bevorzugte aromatische Amine sind Nitrobenzol und/oder Dinitrotoluol, so dass durch deren Hydrierung Anilin beziehungsweise Toluylendiamin erhalten wird.

In einer weiteren Ausführungsform des erfindungsgemäßen Verfahrens ist im Reaktor der Katalysator in einem festen Katalysatorbett angeordnet.

In einer weiteren Ausführungsform des erfindungsgemäßen Verfahrens wird nach Schritt D1) und/oder D2) und vor einem erneuten Zuführen von aromatischen Nitroverbindungen und Wasserstoff in den Reaktor unter Kontaktierung des Hydrierkatalysators das Innere des Reaktors und/oder das Innere von mit dem Reaktor fluidisch verbundenen Anlagenteilen auf eine Temperatur von mehr als 58 °C beheizt. Oberhalb dieser Temperatur zerfällt Ammoniumcarbonat, welches sich im Laufe der Hydrierreaktionen aus Nebenprodukten bilden kann, zu CO₂, NH₃ und Wasser. Das Aufheizen hilft somit ebenfalls, Verstopfungen in der Reaktoranlage und insbesondere im Abgassystem zu vermeiden.

Anhand der nachfolgenden Beispiele soll die vorliegende Erfindung näher beschrieben werden, ohne jedoch darauf beschränkt zu sein.

In den Hydrierreaktionen wurde Wasserstoff im Überschuss eingesetzt (Molverhältnis Wasserstoff/Nitrobenzol = 6:1). Der überschüssige Wasserstoff wurde in der Kondensation der Reaktionsprodukte zurückgewonnen und wieder in die Reaktion eingebracht (Kreisgaswasserstoff). Verbrauchter Wasserstoff wurde durch frischen ergänzt (FrischWasserstoff). Zur Kreisführung des zurückgewonnenen Wasserstoffs diente ein Flüssigkeitsringverdichter. Der Kreisgas-Wasserstoff enthielt gasförmige Verunreinigungen. Um eine Anreicherung dieser Stoffe im Kreislauf zu vermeiden, wurde ein Teilstrom ausgeschleust. Dieser wurde über ein Abgassystem der thermischen Abluftreinigung zugeführt. Die auskondensierten Reaktionsprodukte wurden in einem Flüssig-Gas-Abscheider (Produktabscheider) abgetrennt und in einem Kondensatbehälter gesammelt.

Nach Vorwärmung mittels Dampf wurde Nitrobenzol im Wasserstoff-Strom verdampft und dabei mit dem Wasserstoff vermischt. Dieses Gemisch wurde dann dem Kreisgas vor dem Reaktor zugeführt. Die Reaktoren waren konstruktiv Röhrenbündel-Wärmetauscher. In den Rohren befand sich der Katalysator. Hier erfolgt die Hydrierung des Nitrobenzols. Es entstand ein Rohanilin/Wasser-Gemisch, das die Reaktoren zusammen mit überschüssigem Wasserstoff gasförmig verließ und der Kondensation zugeleitet wird. Die Temperierung des Rohrbündelreaktors erfolgte durch einen Öl-Kreislauf auf 240 °C. Der Anlagendruck nach dem Reaktor betrug 1,2 bar(a).

Die Einsatzstoffe Nitrobenzol und Wasserstoff waren kommerziell erhältliche Ware und hatten die handelsüblichen Spezifikationen.

### Beispiel 1: Regenerierung

Nach einem Produktionszyklus wurde gebrauchter Katalysator mit geringer Restaktivität, der zur Hydrierung von Nitrobenzol zu Anilin verwendet wurde, durch eine Abbrennstufe regeneriert. Dazu wurde der Reaktionsraum zunächst mit Stickstoff inertisiert und dann bei ca. 270 °C mit einem Luftstrom beaufschlagt, um Verkokungen abzubrennen. Dies wurde so lange durchgeführt, bis keine Wänneabgabe mehr zu erkennen war und der CO₂-Gehalt im Abgasstrom auf weniger als 0,2 % gefallen war (bestimmt durch IR-Photometrie).

### Beispiel 2 (Vergleichsbeispiel): Anfahren und Betrieb der Hydrierung von Nitrobenzol in einer Anlage nach einer Regeneration des Hydrierkatalysators.

Nach der Regeneration wurden die Reaktionsapparate und deren Peripherie des Reaktionssystems mit Stickstoff inertisiert. Dazu wurde Stickstoff in das Kreisgassystem eingeleitet und gleichzeitig ein Purge aus dem Kreisgas ins Abgas gefahren, bis die Sauerstoffkonzentration im Kreisgas unterhalb von 0,5% lag. Dann wurde begonnen, auf die gleiche Art Wasserstoff im Kreisgassystem anzureichern, bevor letztlich die Nitrobenzol-Zufuhr und damit die Hydrierreaktion gestartet wurden. Die Reaktion musste nach 4 Tagen unterbrochen werden, weil es zu Verstopfungen im Abgassystem gekommen war. Bei der Reinigung des Abgassystems wurde ein weißer Niederschlag gefunden. Die Elementaranalyse des Niederschlags war im Einklang mit der Zusammensetzung von Ammoniumcarbonat.

### Beispiel 3 (erfindungsgemäßes Beispiel): Anfahren und Betrieb der Hydrierung von Nitrobenzol in einer Anlage nach einer Regeneration des Hydrierkatalysators.

Nach der Regenerierung wurden die Reaktionsapparate und deren Peripherie wie das Abgassystem inertisiert. Das Wasser, das als Betriebsflüssigkeit im Flüssigringverdichter des Wasserstoffkreisgasverdichters dient, wurde durch frisches Wasser ersetzt. Die Anlage wurde wie in Beispiel 1 beschrieben angefahren. Eine Verstopfung des Abgassystems wurde nicht beobachtet, auch bei einer späteren Inspektion wurden keine Ablagerungen gefunden.

## Patentansprüche

1. Verfahren zur Herstellung von aromatischen Aminen durch Hydrierung von aromatischen Nitroverbindungen, umfassend die Schritte:
A) Bereitstellen eines Reaktors mit hierin enthaltenem Hydrierkatalysator;
B) Zuführen von aromatischen Nitroverbindungen und Wasserstoff in den Reaktor unter Kontaktierung des Hydrierkatalysators, wobei wenigstens der Wasserstoff mittels eines Verdichters in den Reaktor zugeführt wird und der Verdichter Betriebsflüssigkeit enthält, welche den Wasserstoff zumindest teilweise kontaktiert;
C) Regenerieren des Hydrierkatalysators durch Erwärmen und Kontaktieren mit Sauerstoff; **dadurch gekennzeichnet, dass** nach Schritt C) die Schritte D1) und/oder D2) durchgeführt werden:
D1) Zumindest teilweises Entfernen von im Reaktor und/oder in stromabwärts mit dem Reaktor fluidisch verbundenen Anlagenteilen befindlichen Flüssigkeiten, welche zumindest teilweise während Schritt C) im Reaktor und/oder in diesen Anlagenteilen anwesend waren;
D2) Zumindest teilweises Austauschen der Betriebsflüssigkeit des Verdichters, welche während Schritt C) im Verdichter und/oder in mit dem Verdichter verbundenen Rezirkulierungseinrichtungen anwesend war, gegen Betriebsflüssigkeit, welche während Schritt C) nicht im Verdichter und/oder in mit dem Verdichter verbundenen Rezirkulierungseinrichtungen anwesend war.

2. Verfahren gemäß Anspruch 1, wobei der Verdichter ein Flüssigkeitsringverdichter ist.

3. Verfahren gemäß Anspruch 1, wobei die Betriebsflüssigkeit im Verdichter Wasser oder eine wässrige Lösung mit mehr als 90 Gewichts-% Wasser ist.

4. Verfahren gemäß Anspruch 1, wobei nach Schritt C) der Reaktor zusätzlich mit einem Inertgas befüllt oder gespült wird.

5. Verfahren gemäß Anspruch 1, wobei Schritt D1) durchgeführt wird, indem der Reaktor und/oder stromabwärts mit dem Reaktor fluidisch verbundenen Anlagenteile mit Wasser und/oder einer wässrigen Lösung mit mehr als 90 Gewichts-% Wasser gespült wird.

6. Verfahren gemäß Anspruch 1, wobei in Schritt B) das molare Verhältnis von Wasserstoff zu Nitrogruppen der aromatischen Nitroverbindungen ≥ 3:1 bis ≤ 100:1 beträgt.

7. Verfahren gemäß Anspruch 1, wobei in Schritt B) aromatische Nitroverbindungen gemäß der allgemeinen Formel (I) eingesetzt werden: 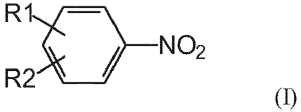 in der R1 und R2 unabhängig voneinander Wasserstoff, Methyl oder Ethyl bedeuten, wobei R2 weiterhin auch NO₂ bedeuten kann.

8. Verfahren gemäß Anspruch 1, wobei die aromatischen Nitroverbindungen in Schritt B) Nitrobenzol und/oder Dinitrotoluol sind.

9. Verfahren gemäß Anspruch 1, wobei im Reaktor der Katalysator in einem festen Katalysatorbett angeordnet ist.

10. Verfahren gemäß Anspruch 1, wobei nach Schritt D1) und/oder D2) und vor einem erneuten Zuführen von aromatischen Nitroverbindungen und Wasserstoff in den Reaktor unter Kontaktierung des Hydrierkatalysators das Innere des Reaktors und/oder das Innere von mit dem Reaktor fluidisch verbundenen Anlagenteilen auf eine Temperatur von mehr als 58 °C beheizt wird.

## Claims

1. Process for preparing aromatic amines by hydrogenation of aromatic nitro compounds, which comprises the steps:
A) provision of a reactor with hydrogenation catalyst present therein;
B) introduction of aromatic nitro compounds and hydrogen into the reactor with contacting of the hydrogenation catalyst, with at least the hydrogen being fed into the reactor by means of a compressor and the compressor containing operating liquid which at least partially contacts the hydrogen;
C) regeneration of the hydrogenation catalyst by heating and contacting with oxygen;
**characterized in that** the steps D1) and/or D2):
D1) at least partial removal of liquids which are present in the reactor and/or in plant parts fluidically connected downstream to the reactor and were at least partly present in the reactor and/or in these plant parts during step C);
D2) at least partial replacement of the operating liquid of the compressor which was present in the compressor and/or in recirculation facilities connected to the compressor during step C) by operating liquid which was not present in the compressor and/or in recirculation facilities connected to the compressor during step C);
are carried out after step C).

2. Process according to Claim 1, wherein the compressor is a liquid ring compressor.

3. Process according to Claim 1, wherein the operating liquid in the compressor is water or an aqueous solution containing more than 90% by weight of water.

4. Process according to Claim 1, wherein the reactor is additionally filled or flushed with an inert gas after step C).

5. Process according to Claim 1, wherein step D1) is carried out by flushing the reactor and/or plant parts fluidically connected downstream to the reactor with water and/or an aqueous solution containing more than 90% by weight of water.

6. Process according to Claim 1, wherein the molar ratio of hydrogen to nitro groups of the aromatic nitro compounds in step B) is from ≥ 3:1 to ≤ 100:1.

7. Process according to Claim 1, wherein aromatic nitro compounds having the general formula (I): 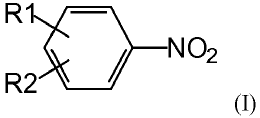 where R1 and R2 are each, independently of one another, hydrogen, methyl or ethyl and R2 can also be NO₂, are used in step B).

8. Process according to Claim 1, wherein the aromatic nitro compounds in step B) are nitrobenzene and/or dinitrotoluene.

9. Process according to Claim 1, wherein the catalyst in the reactor is arranged in a fixed catalyst bed.

10. Process according to Claim 1, wherein the interior of the reactor and/or the interior of plant parts fluidically connected to the reactor is/are heated to a temperature of more than 58°C after step D1) and/or D2) and before renewed introduction of aromatic nitro compounds and hydrogen into the reactor with contacting of the hydrogenation catalyst.

## Revendications

1. Procédé de fabrication d'amines aromatiques par hydrogénation de composés nitro aromatiques, comprenant les étapes suivantes :
A) la préparation d'un réacteur avec un catalyseur d'hydrogénation contenu dans celui-ci ;
B) l'introduction de composés nitro aromatiques et d'hydrogène dans le réacteur avec mise en contact du catalyseur d'hydrogénation, au moins l'hydrogène étant introduit dans le réacteur au moyen d'un compresseur, et le compresseur contenant un liquide d'exploitation, qui contacte au moins en partie l'hydrogène ;
C) la régénération du catalyseur d'hydrogénation par chauffage et mise en contact avec de l'oxygène ;
**caractérisé en ce que** les étapes D1) et/ou D2) sont réalisées après l'étape C) :
D1) l'élimination au moins partielle de liquides se trouvant dans le réacteur et/ou dans des parties de l'installation reliées fluidiquement en aval du réacteur, qui étaient présents au moins en partie pendant l'étape C) dans le réacteur et/ou dans ces parties de l'installation ;
D2) le remplacement au moins partiel du liquide d'exploitation du compresseur qui était présent pendant l'étape C) dans le compresseur et/ou dans des dispositifs de recyclage reliés avec le compresseur, par un liquide d'exploitation qui n'était pas présent pendant l'étape C) dans le compresseur et/ou dans des dispositifs de recyclage reliés avec le compresseur.

2. Procédé selon la revendication 1, dans lequel le compresseur est un compresseur à anneau liquide.

3. Procédé selon la revendication 1, dans lequel le liquide d'exploitation dans le compresseur est de l'eau ou une solution aqueuse contenant plus de 90 % en poids d'eau.

4. Procédé selon la revendication 1, dans lequel le réacteur est en outre rempli ou rincé avec un gaz inerte après l'étape C).

5. Procédé selon la revendication 1, dans lequel l'étape D1) est réalisée par rinçage du réacteur et/ou de parties de l'installation reliées fluidiquement en aval du réacteur avec de l'eau et/ou une solution aqueuse contenant plus de 90 % en poids d'eau.

6. Procédé selon la revendication 1, dans lequel, à l'étape B), le rapport molaire entre l'hydrogène et les groupes nitro des composés nitro aromatiques est de ≥ 3:1 à ≤ 100:1.

7. Procédé selon la revendication 1, dans lequel, à l'étape B), des composés nitro aromatiques selon la formule générale (I) sont utilisés : 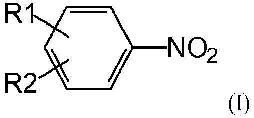 dans laquelle R1 et R2 signifient indépendamment l'un de l'autre hydrogène, méthyle ou éthyle, R2 pouvant également signifie NO₂.

8. Procédé selon la revendication 1, dans lequel les composés nitro aromatique à l'étape B) sont le nitrobenzène et/ou le dinitrotoluène.

9. Procédé selon la revendication 1, dans lequel le catalyseur est agencé dans un lit catalytique fixe dans le réacteur.

10. Procédé selon la revendication 1, dans lequel, après l'étape D1) et/ou D2) et avant une nouvelle introduction de composés nitro aromatiques et d'hydrogène dans le réacteur avec mise en contact du catalyseur d'hydrogénation, l'intérieur du réacteur et/ou l'intérieur de parties de l'installation reliées fluidiquement avec le réacteur est porté à une température de plus de 58 C.
